# EUROPEAN PATENT SPECIFICATION

(11) **EP 2 450 453 B1**
(45) Date of publication and mention of the grant of the patent: **31.05.2017**
(21) Application number: 10793650.2
(22) Date of filing: 02.07.2010
(51) Int. Cl.: C12Q 1/68

(54) **METHOD FOR THE REPLICATION, AMPLIFICATION OR SEQUENCING OF A DNA TEMPLATE**
VERFAHREN ZUR REPLIKATION, VERSTÄRKUNG ODER SEQUENZIERUNG EINER DNA-VORLAGE
MÉTHODE DE RÉPLICATION, D'AMPLIFICATION OU DE SÉQUENÇAGE D'UNE ADN MATRICE

(30) Priority: 02.07.2009 ES 200930412
(43) Date of publication of application: 09.05.2012
(73) Proprietor: Consejo Superior de Investigaciones Cientificas (CSIC), 28006 Madrid (ES)
(72) Inventor: SALAS FALGUERAS, Margarita, E-28049 Madrid (ES); DE VEGA JOSÉ, Miguel, E-28049 Madrid (ES); LAZARO BOLOS, José Mª, E-28049 Madrid (ES); BLANCO DAVILA, Luis, E-28049 Madrid (ES); MENCIA CABALLERO, Mario, E-28049 Madrid (ES)
(74) Representative: ABG Patentes, S.L.
(86) International application number: PCT/ES2010/070456
(87) International publication number: WO 2011/000998

(56) References cited:
- WO-A1-2004/072304
- WO-A2-01/25483
- US-A- 5 198 543
- SHOAIB, M. ET AL.: 'Multiple displacement amplification for complex mixtures of DNA fragments.' BMC GENOMICS. vol. 9, no. 1, September 2008, pages 415 - 429, XP021042155
- HART, M. C. ET AL.: 'Large subunit ribosomal RNA gene variation and sequence heterogeneity of Dinophysis (Dinophyceae) species from Scottish coastal waters.' HARMFUL ALGAE. vol. 6, no. 2, January 2007, pages 271 - 287, XP005857797
- BLANCO, L. ET AL.: 'Effect of NH4+ ions on phi29 DNA-protein p3 replication: formation of a complex between the terminal protein and the DNA polymerase.' JOURNAL OF VIROLOGY. vol. 61, no. 12, December 1987, pages 3983 - 3991, XP008150106
- BLASCO, M. A. ET AL.: 'Structural and functional analysis of temperature-sensitive mutants of the phage phi29 DNA polymerase.' NUCLEIC ACIDS RESEARCH vol. 18, no. 16, August 1990, pages 4763 - 4770, XP002386962 & DATABASE UNIPROT DATABASE. [Online] 16 December 2008 Database accession no. Q38545

### SEQUENCE LISTING

<110> Consejo Superior de Investigaciones Cientificas
<120> Method for the Replication, Amplification or Sequencing of **a** DNA Template
<130> ES1641.386.2
<140> EP 10793650.2
   <141> 2010-07-02
<150> ES P200930412
   <151> 2009-07-02
<160> 1
<170> PatentIn version 3.5
<210> 1
   <211> 572
   <212> PRT
   <213> Bacteriophage phi-29
<400> 1
   Met Pro Arg Lys Met Tyr Ser Cys Asp Phe Glu Thr Thr Thr Lys Val
   1               5                   10                  15
   Glu Asp Cys Arg Val Trp Ala Tyr Gly Tyr Met Asn Ile Glu Asp His
               20                  25                  30
   Ser Glu Tyr Lys Ile Gly Asn Ser Leu Asp Glu Phe Met Ala Trp Val
           35                  40                  45
   Leu Lys Val Gln Ala Asp Leu Tyr Phe His Asn Leu Lys Phe Asp Gly
       50                  55                  60
   Ala Phe Ile Ile Asn Trp Leu Glu Arg Asn Gly Phe Lys Trp Ser Ala
   65                  70                  75                  80
   Asp Gly Leu Pro Asn Thr Tyr Asn Thr Ile Ile Ser Arg Met Gly Gln
                   85                  90                  95
   Trp Tyr Met Ile Asp Ile Cys Leu Gly Tyr Lys Gly Lys Arg Lys Ile
               100                 105                 110
   His Thr Val Ile Tyr Asp Ser Leu Lys Lys Leu Pro Phe Pro Val Lys
           115                 120                 125
   Lys Ile Ala Lys Asp Phe Lys Leu Thr Val Leu Lys Gly Asp Ile Asp
       130                 135                 140
   Tyr His Lys Glu Arg Pro Val Gly Tyr Lys Ile Thr Pro Glu Glu Tyr
   145                 150                 155                 160
   Ala Tyr Ile Lys Asn Asp Ile Gln Ile Ile Ala Glu Arg Leu Leu Ile
                   165                 170                 175
   Gln Phe Lys Gln Gly Leu Asp Arg Met Thr Ala Gly Ser Asp Ser Leu
               180                 185                 190
   Lys Gly Phe Lys Asp Ile Ile Thr Thr Lys Lys Phe Lys Lys Val Phe
           195                 200                 205
   Pro Thr Leu Ser Leu Gly Leu Asp Lys Glu Val Arg Tyr Ala Tyr Arg
       210                 215                 220
   Gly Gly Phe Thr Trp Leu Asn Asp Arg Phe Lys Glu Lys Glu Ile Gly
   225                 230                 235                 240
   Glu Gly Met Val Phe Asp Val Asn Ser Leu Tyr Pro Ala Gln Met Tyr
                   245                 250                 255
   Ser Arg Leu Leu Pro Tyr Gly Glu Pro Ile Val Phe Glu Gly Lys Tyr
               260                 265                 270
   Val Trp Asp Glu Asp Tyr Pro Leu His Ile Gln His Ile Arg Cys Glu
           275                 280                 285
   Phe Glu Leu Lys Glu Gly Tyr Ile Pro Thr Ile Gln Ile Lys Arg Ser
       290                 295                 300
   Arg Phe Tyr Lys Gly Asn Glu Tyr Leu Lys Ser Ser Gly Gly Glu Ile
   305                 310                 315                 320
   Ala Asp Leu Trp Leu Ser Asn Val Asp Leu Glu Leu Met Lys Glu His
                   325                 330                 335
   Tyr Asp Leu Tyr Asn Val Glu Tyr Ile Ser Gly Leu Lys Phe Lys Ala
               340                 345                 350
   Thr Thr Gly Leu Phe Lys Asp Phe Ile Asp Lys Trp Thr Tyr Ile Lys
           355                 360                 365
   Thr Thr Ser Glu Gly Ala Ile Lys Gln Leu Ala Lys Leu Met Leu Asn
       370                 375                 380
   Ser Leu Tyr Gly Lys Phe Ala Ser Asn Pro Asp Val Thr Gly Lys Val
   385                 390                 395                 400
   Pro Tyr Leu Lys Glu Asn Gly Ala Leu Gly Phe Arg Leu Gly Glu Glu
                   405                 410                 415
   Glu Thr Lys Asp Pro Val Tyr Thr Pro Met Gly Val Phe Ile Thr Ala
               420                 425                 430
   Trp Ala Arg Tyr Thr Thr Ile Thr Ala Ala Gln Ala Cys Tyr Asp Arg
           435                 440                 445
   Ile Ile Tyr Cys Asp Thr Asp Ser Ile His Leu Thr Gly Thr Glu Ile
       450                 455                 460
   Pro Asp Val Ile Lys Asp Ile Val Asp Pro Lys Lys Leu Gly Tyr Trp
   465                 470                 475                 480
   Ala His Glu Ser Thr Phe Lys Arg Val Lys Tyr Leu Arg Gln Lys Thr
                   485                 490                 495
   Tyr Ile Gln Asp Ile Tyr Met Lys Glu Val Asp Gly Lys Leu Val Glu
               500                 505                 510
   Gly Ser Pro Asp Asp Tyr Thr Asp Ile Lys Phe Ser Val Lys Cys Ala
           515                 520                 525
   Gly Met Thr Asp Lys Ile Lys Lys Glu Val Thr Phe Glu Asn Phe Lys
       530                 535                 540
   Val Gly Phe Ser Arg Lys Met Lys Pro Lys Pro Val Gln Val Pro Gly
   545                 550                 555                 560
   Gly Val Val Leu Val Asp Asp Thr Phe Thr Ile Lys
                   565                 570

## Description

The present invention is encompassed within the biotechnology field. Specifically, it relates to a method for replicating, amplifying or sequencing a template DNA with a φ29 type DNA polymerase.

### Prior State of the Art

The only enzyme required by the bacteriophage φ29 to replicate its genome is its DNA polymerase, a 66 KDa monomeric protein capable of catalyzing both the initiation of the replication and the elongation of the synthesized strand. For the initiation, this polymerase is bound to a protein known as "terminal" (TP), recognizes the end of the φ29 DNA and catalyzes the formation of a TP-dAMP covalent complex. After the polymerization of 10 nucleotides, the DNA polymerase/TP heterodimer disassociates and the elongation of the strand coming from DNA is carried out.

Replicative DNA polymerases require the interaction with accessory proteins which stabilize the binding between the enzyme and the DNA (Kuriyan and O'Donnell. J Mol Biol. 1993; 234: 915-925). On the other hand, said DNA polymerases need to couple the polymerization upon the detachment of the DNA strand which is not being copied for which they require the functional association thereof to helicase type proteins. In this sense, the DNA polymerase of the bacteriophage φ29 has various intrinsic functional characteristics making it unique:
a) High processivity (defined as the number of nucleotides incorporated by binding event).
b) High strand detachment capacity which allows replicating the genome of said bacteriophage in the absence of helicase type accessory proteins. These two characteristics, processivity and strand detachment allow the φ29 DNA polymerase to be capable of synthesizing DNA strands of more than 70 kb in length (Blanco et al. J Biol Chem. 1989; 264: 8935-8940).
c) High accuracy in the insertion of nucleotides in the new strand (Esteban et al. J Biol Chem. 1993; 268: 2719-2726).

All these characteristics have led to the development of a great variety of isothermal process (at constant temperature) protocols for amplifying double stranded DNA (dsDNA) based on the use of this polymerase. In a simple configuration, the capacity of the φ29 DNA polymerase to use circular single stranded DNA (ssDNA) allows amplifying DNA by the rolling circle method (or RCA - rolling-circle amplification), producing ssDNA molecules of great length and containing more than 10 copies of the circular template (Blanco et al. J Biol Chem. 1989; 264: 8935-8940; US5001050, US5198543 and US5576204). In the process for amplifying dsDNA developed by Amersham Biosciences / Molecular Staging (Dean et al. Genome Res. 2001; 11: 1095-1099; Dean et al. Proc Natl Acad Sci USA. 2002; 99: 5261-5266), the combination of the use of the φ29 DNA polymerase with the use of hexamers (hexa-nucleotides) random sequence primers allows obtaining amplification factors of 10⁴-10⁶ starting from picograms of circular plasmid DNA [Templiphi™ of GE Healthcare] or from 10 nanograms of Genomic DNA [Genomiphi™ of GE Healthcare and Repli-G^{®} of Qiagen]. The products produced are of high quality and can be digested or sequenced directly without the need of prior purification, it has been demonstrated that the φ29 DNA polymerase is the most robust enzyme for this purpose. The common buffer for carrying out the amplification reactions with the φ29 DNA polymerase contains tris-HCl (pH 7.5) plus different concentrations (in the millimolar order) of NaCl or KCl and MgCl₂ (US20030207267). Document WO2004/072304 discloses an amplification method of a DNA input in the nanogram range using φ29 DNA polymerase and a buffer containing 5 mM ammonium sulfate. However, in spite of the satisfactoriness of these protocols in very diverse situations, the development of other protocols which allow starting from lesser DNA amounts is a growing need.

### Summary of the Invention

The present invention relates to a method for replicating, amplifying or sequencing a template DNA with a φ29 type DNA polymerase. A kit for carrying out said method is also disclosed.

The phage φ29 DNA polymerase has several characteristics of great interest for amplifying DNA such as: a high processivity without the need of the participation of any accessory protein and a high strand detachment capacity allowing it to replicate the genome of said bacteriophage in a single binding event to the DNA, as well as a high accuracy in the insertion of nucleotides in the new strand. These characteristics have led to the development of a great variety of protocols for the isothermal amplification of DNA based on the use of this polymerase which allow obtaining products of high quality that can be digested or sequenced directly without the need of prior purification. However, there is a need for protocols which allow the amplification of DNA from lesser amounts thereof. The present invention responds to this need by means of developing a method for amplifying DNA which significantly improves the specificity and the yield of the reaction.

In the examples of this patent, it is shown that the simultaneous addition of polyoxyethylenated sorbitan monolaurate (Tween^{®} 20) and an ammonium salt to the buffer commonly used for the amplification with the φ29 DNA polymerase, on the one hand, prevents the non-specific DNA amplification and, on the other hand allows the detectable and specific amplification from limited amounts of 0.1 femtograms (fg) of plasmid DNA and 10 fg of genomic DNA as template.

A first aspect of the present invention relates to a method for replicating, amplifying or sequencing a template DNA which comprises contacting said DNA with a reaction mixture comprising at least:
a) a φ29 type DNA polymerase,
b) polyoxyethylenated sorbitan monolaurate, wherein the polyoxyethylenated sorbitan monolaurate is in a proportion between 0.006% and 0.05% of the total volume of the reaction,
c) an ammonium salt, wherein the ammonium salt is ammonium sulfate at a concentration between 30 mM and 60 mM, ammonium chloride at a concentration between 60 mM and 120 mM or ammonium acetate at a concentration between 60 mM and 120 mM
d) a buffer,
e) magnesium chloride,
f) a primer, and
g) nucleoside triphosphates.

A preferred embodiment of this aspect of the invention relates to a method for replicating, amplifying or sequencing a template DNA which comprises contacting said DNA with a reaction mixture comprising the aforementioned elements (a)-(g) and further comprising a potassium salt. Preferably, said potassium salt is potassium chloride or potassium acetate.

As used in the present description, the term "DNA polymerase" relates to an enzyme capable of catalyzing the polymerization of deoxynucleoside triphosphates. Generally, the enzyme initiates the synthesis in the 3' end of a primer hybridized with a template DNA sequence and proceeds towards the 5' end of the template DNA strand.

As used in the present invention, the term "φ29 type DNA polymerase" relates to any DNA polymerase containing TPR1 and TPR2 subdomains in its polymerization domain providing the polymerase with the capacity of coupling the processive polymerization to the strand detachment. Examples of φ29 type DNA polymerases that can be used in the present invention are selected from the list comprising the DNA polymerases isolated from the following phages: φ29, Cp-1, PRD-1, φ15, φ21, PZE, PZA, Nf, M2Y, B103, GA-1, SF5, Cp-5, Cp-7, PR4, PR5, PR722, L17 or Acidianus Bottle-shaped virus (ABV).

In a preferred embodiment of the method for replicating, amplifying or sequencing of the invention, the φ29 type DNA polymerase is selected from the DNA polymerases isolated from the following phages: φ29, Cp-1, PRD-1, φ15, φ21, PZE, PZA, Nf, M2Y, B103, GA-1, SF5, Cp-5, Cp-7, PR4, PR5, PR722, L17 or Acidianus Bottle-shaped virus (ABV).

In a preferred embodiment of the method for replicating, amplifying or sequencing of the invention, the φ29 type DNA polymerase has an amino acid sequence having an identity of at least 80% with SEQ ID NO: 1. In a more preferred embodiment, the φ29 type DNA polymerase has an amino acid sequence having an identity of at least 90% with SEQ ID NO: 1. In a still more preferred embodiment, the φ29 type DNA polymerase has the amino acid sequence SEQ ID NO: 1.

The exonuclease domain of the φ29 type DNA polymerases is known and can be modified to reduce the exonuclease activity retaining a high processivity and strand detachment capacity. These modified DNA polymerases are especially useful for sequencing large molecules.

In a preferred embodiment of the method for replicating, amplifying or sequencing of the invention, the φ29 type DNA polymerase has a modification in the exonuclease domain, wherein said modified DNA polymerase has less than 10% of exonuclease activity than the corresponding naturally occurring DNA polymerase or "wild type". In a more preferred embodiment, the modified φ29 type DNA polymerase has less than 1% of exonuclease activity than the corresponding naturally occurring DNA polymerase. In a still more preferred embodiment, the modified φ29 type DNA polymerase lacks detectable exonuclease activity with respect to the corresponding naturally occurring DNA polymerase.

In a preferred embodiment of the method for replicating, amplifying or sequencing of the invention, the φ29 type DNA polymerase (natural or modified in the exonuclease domain) is at a concentration between 5 nM and 75 nM. In a more preferred embodiment, the φ29 type DNA polymerase (natural or modified in the exonuclease domain) is at a concentration between 25 nM and 60 nM. In a still more preferred embodiment, the φ29 type DNA polymerase (natural or modified in the exonuclease domain) is at a concentration of approximately 50 nM.

In a preferred embodiment of the method for replicating, amplifying or sequencing of the invention, the polyoxyethylenated sorbitan monolaurate (Tween^{®} 20) is at a concentration between 0.003% and 0.1% of the total volume of the reaction. In a more preferred embodiment, the polyoxyethylenated sorbitan monolaurate is in a proportion between 0.006% and 0.05% of the total volume of the reaction. In a still more preferred embodiment, the polyoxyethylenated sorbitan monolaurate is in a proportion between 0.01% and 0.03% of the total volume of the reaction. In a still more preferred embodiment, the polyoxyethylenated sorbitan monolaurate is in a proportion of approximately 0.025% of the total volume of the reaction. "Total volume of the reaction" is understood as the resulting volume after the addition of the template DNA to the reaction mixture.

In the method for replicating, amplifying or sequencing of the invention, the ammonium salt is selected from the list comprising: ammonium sulfate at a concentration between 30 mM and 60 mM, ammonium chloride ammonium chloride at a concentration between 60 mM and 120 mM or ammonium acetate at a concentration between 60 mM and 120 mM.

In a preferred embodiment of the method for replicating, amplifying or sequencing of the invention, the ammonium salt is ammonium sulfate at a concentration between 30 mM and 60 mM. In a still more preferred embodiment, the ammonium sulfate is at a concentration between 40 mM and 50 mM. In a still more preferred embodiment, the ammonium sulfate is at a concentration of approximately 45 mM.

In a preferred embodiment of the method for replicating, amplifying or sequencing of the invention, the ammonium salt is ammonium chloride at a concentration between 60 mM and 120 mM. In a still more preferred embodiment, the ammonium chloride is at a concentration between 80 mM and 100 mM. In a still more preferred embodiment, the ammonium chloride is at a concentration of approximately 90 mM.

In a preferred embodiment of the method for replicating, amplifying or sequencing of the invention, the ammonium salt is ammonium acetate at a concentration between 60 mM and 120 mM. In a still more preferred embodiment, the ammonium acetate is at a concentration between 80 mM and 100 mM. In a still more preferred embodiment, the ammonium acetate is at a concentration of approximately 90 mM.

In a preferred embodiment of the method for replicating, amplifying or sequencing of the invention, the buffer is at a pH between 7.0 and 8.5. In a more preferred embodiment, the buffer is at a pH between 7.2 and 8. In a still more preferred embodiment, the buffer is at a pH of approximately 7.5.

In a preferred embodiment of the method for replicating, amplifying or sequencing of the invention, the buffer is tris-hydrochloric, tris-acetic or HEPES. In a more preferred embodiment of the method for replicating, amplifying or sequencing of the invention, the buffer tris-hydrochloric, tris-acetic or HEPES is at a pH between 7.0 and 8.5. In a still more preferred embodiment, the buffer tris-hydrochloric, tris-acetic or HEPES is at a pH between 7.2 and 8. In a still more preferred embodiment, the buffer tris-hydrochloric, tris-acetic or HEPES is at a pH of approximately 7.5.

In a preferred embodiment of this aspect of the method for replicating, amplifying or sequencing of the invention, the buffer tris-hydrochloric, tris-acetic or HEPES is at a concentration between 25 mM and 50 mM. In a more preferred embodiment, the buffer tris-hydrochloric, tris-acetic or HEPES is at a concentration between 30 mM and 45 mM. In a still more preferred embodiment, the buffer tris-hydrochloric, tris-acetic or HEPES is at a concentration of approximately 40 mM.

In a preferred embodiment of the method for replicating, amplifying or sequencing of the invention, the potassium chloride or the potassium acetate is at a concentration between 30 mM and 70 mM. In a more preferred embodiment, the potassium chloride or the potassium acetate is at a concentration between 40 mM and 60 mM. In a still more preferred embodiment, the potassium chloride or the potassium acetate is at a concentration of approximately 50 mM.

In a preferred embodiment of the method for replicating, amplifying or sequencing of the invention, the magnesium chloride is at a concentration between 2 mM and 20 mM. In a more preferred embodiment, the magnesium chloride is at a concentration between 5 mM and 15 mM. In a still more preferred embodiment, the magnesium chloride is approximately 10 mM.

In a preferred embodiment of the method for replicating, amplifying or sequencing of the invention, the polyoxyethylenated sorbitan monolaurate is in a proportion between 0.01% and 0.03% of the total volume, the ammonium sulfate is at a concentration between 40 mM and 50 mM, the buffer tris-hydrochloric, tris-acetic or HEPES is at a concentration between 30 mM and 45 mM and at a pH between 7.2 and 8.0, the magnesium chloride is at a concentration between 5 mM and 15 mM and the potassium chloride or the potassium acetate is at a concentration between 40 mM and 60 mM.

In a preferred embodiment of the method for replicating, amplifying or sequencing of the invention, the polyoxyethylenated sorbitan monolaurate is in a concentration of 0.025% of the total volume, the ammonium sulfate is at a concentration of 45 mM, the buffer tris-hydrochloric, tris-acetic or HEPES is at a concentration of 40 mM and at a pH of 7.5, the magnesium chloride is at a concentration of 10 mM and the potassium chloride or the potassium acetate is at a concentration of 50 mM.

In a preferred embodiment of the method for replicating, amplifying or sequencing of the invention, the polyoxyethylenated sorbitan monolaurate is in a proportion between 0.01% and 0.03% of the total volume, the ammonium chloride is at a concentration between 80 mM and 100 mM, the buffer tris-hydrochloric, tris-acetic or HEPES is at a concentration between 30 mM and 45 mM and at a pH between 7.2 and 8.0, the magnesium chloride is at a concentration between 5 mM and 15 mM and the potassium chloride or the potassium acetate is at a concentration between 40 mM and 60 mM.

In a more preferred embodiment of the method for replicating, amplifying or sequencing of the invention, the polyoxyethylenated sorbitan monolaurate is in a concentration of 0.025% of the total volume, the ammonium chloride is at a concentration of 90 mM, the buffer tris-hydrochloric, tris-acetic or HEPES is at a concentration of 40 mM and at a pH of 7.5, the magnesium chloride is at a concentration of 10 mM and the potassium chloride or the potassium acetate is at a concentration of 50 mM.

In a preferred embodiment of the method for replicating, amplifying or sequencing of the invention, the polyoxyethylenated sorbitan monolaurate is in a proportion between 0.01% and 0.03% of the total volume, the ammonium acetate is at a concentration of between 80 mM and 100 mM, the buffer tris-hydrochloric, tris-acetic or HEPES is at a concentration between 30 mM and 45 mM and at a pH between 7.2 and 8.0, the magnesium chloride is at a concentration between 5 mM and 15 mM and the potassium chloride or the potassium acetate is at a concentration between 40 mM and 60 mM.

In a more preferred embodiment of the method for replicating, amplifying or sequencing of the invention, the polyoxyethylenated sorbitan monolaurate is in a concentration of 0.025% of the total volume, the ammonium acetate is at a concentration of 90 mM, the buffer tris-hydrochloric, tris-acetic or HEPES is at a concentration of 40 mM and at a pH of 7.5, the magnesium chloride is at a concentration of 10 mM and the potassium chloride or the potassium acetate is at a concentration of 50 mM.

As used in the present description, the term "replication" relates to the synthesis of a complementary DNA from a template DNA.

As used in the present description, the term "amplification" relates to the increase of the number of copies of a template DNA.

As used in the present description, the term "sequencing" relates to the determination of the order of the nucleotides of a template DNA.

"Contacting" is understood as the fact that the template DNA and the reaction mixture are incubated in primer extension conditions.

As used herein, the term "primer" relates to a oligonucleotide capable of acting as the starting point of the DNA synthesis when it is in primer extension conditions. Preferably, the primer is a deoxyribose oligonucleotide.

The primers can be prepared by means of any suitable method, including for example, but not limited to, the direct chemical synthesis. The primers can be designed to hybridize with specific deoxynucleotide sequences in the template DNA (specific primers) or can be randomly synthetized (arbitrary primers).

As used in the present description, the term "specific primer" relates to a primer the sequence of which is complementary to a specific deoxynucleotide sequence in the template DNA to be amplified.

"Complementary" is understood as the fact that the primer can be hybridized with a region of the template DNA such that it can act as the starting point of the DNA synthesis when it is in primer extension conditions. Preferably, that region has a 100% complementarity with a region of the template DNA. In other words, each nucleotide in the region of complementarity with the primer can form hydrogen bonds with a nucleotide present in the single stranded template. However, those with a normal experience in the field will acknowledge that primers having a region with less than 100% complementarity with respect to the template DNA will function to carry out the method for replicating, amplifying or sequencing of the present invention.

The term "arbitrary primer" relates to a primer the sequence of which is randomly synthesized and which is used to initiate the DNA synthesis in random positions of the template DNA. Generally, in the method for replicating, amplifying or sequencing of the present invention a population of arbitrary primers is used. The term "arbitrary primers" relates to a set of primers with a random sequence and which are used to initiate the DNA synthesis in random positions of the template DNA.

In a preferred embodiment of the method for replicating, amplifying or sequencing of the invention, the primer is specific.

In another preferred embodiment of the method for replicating, amplifying or sequencing of the invention, the primer is arbitrary. Preferably, the arbitrary primer is protected against the action of 3'-5' exonucleases'. And more preferably, the arbitrary primer is an oligonucleotide of 6 nucleotides, "hexanucleotide" or "hexamer" protected against the action of 3'-5' exonucleases.

As used in the present description, the expression "protected against the action of exonucleases" relates to a modified primer such that it is resistant to the nucleolytic degradation by any 3'-5' exonuclease activity present in the DNA polymerase.

In the method for replicating, amplifying or sequencing of the invention, more than one primer can be used, being able to use specific and/or arbitrary primers.

In a preferred embodiment of the method for replicating, amplifying or sequencing of the invention, the primer is at a concentration between 2µM and 100µM. In a more preferred embodiment, the primer is at a concentration between 20µM and 80µM. In a still more preferred embodiment, the primer is at a concentration between 40µm and 60µM. In a still more preferred embodiment, the primer is at a concentration of approximately 50µM.

As used in the present description the term "nucleoside triphosphates" relates to organic molecules formed by the covalent bond of a pentose, a nitrogen base and three phosphate groups.

The term nucleoside triphosphates includes deoxynucleoside triphosphates (dNTPs) such as, for example, but not limited to dATP, dCTP, dITP, dUTP, dGTP, dTTP, or derivatives thereof. Preferably, the deoxynucleoside triphosphates are dATP, dTTP, dGTP and dCTP. Still more preferably, these four dNTPs are in equimolar conditions. In a preferred embodiment of this aspect of the invention, the deoxynucleoside triphosphates are at a concentration between 100µM and 800µM. In a more preferred embodiment, the deoxynucleoside triphosphates are at a concentration between 200µM and 600µM. In a still more preferred embodiment, the deoxynucleoside triphosphates are at a concentration of approximately 500µM.

The term nucleoside triphosphates also includes dideoxynucleoside triphosphates (ddNTPs) such as, for example, but not limited to, ddATP, ddCTP, ddITP, ddUTP, ddGTP, ddTTP, or derivatives thereof.

In some preferred embodiments of the method for replicating, amplifying or sequencing of the invention, at least one nucleoside triphosphate or one primer is labelled by means of techniques well known in the state of the art. The labelled nucleotide can be, for example, a deoxynucleoside triphosphate or a dideoxynucleoside triphosphate. Detectable labels include, for example, radioactive isotopes, fluorescent labels, chemiluminescent labels, bioluminescent labels or enzymatic labels.

As used in the present description, the term "template DNA" relates to a DNA molecule that can serve as a substrate for synthesizing a complementary DNA strand; i.e., it relates to a DNA molecule to be replicated, amplified or sequenced. In a preferred embodiment the template DNA is plasmid DNA. In another preferred embodiment, the template DNA is genomic DNA.

Replicating, amplifying or sequencing of the template DNA is carried out in primer extension conditions. The expression "primer extension conditions" refers to the conditions in which the template DNA-dependent synthesis initiated in a primer can take place.

The template DNA synthesis according to the method for replicating, amplifying or sequencing of the present invention can take place by means of a thermal cycling process or at an essentially constant temperature.

"Isothermal conditions" is understood as essentially constant temperature. Preferably, the template DNA synthesis according to the method for replicating, amplifying or sequencing of the present invention takes place at an essentially constant temperature. More preferably, at an essentially constant temperature between 25 and 40 °C, and still more preferably at approximately 30°C.

A large number of methods allowing DNA amplification are known in the state of the art. Some methods require a thermal cycling process such as, for example, but not limited to the polymerase chain reaction (PCR). Other methods do not require a thermal cycling process, rather they are performed at a essentially constant temperature such as, for example, but not limited to the rolling circle amplification (RCA), the multiple detachment amplification (MDA), the strand displacement amplification (SDA) or the loop mediated amplification (LAMP). The amplification of a template DNA according to the method of the present invention can take place by means of a thermal cycling process or at an essentially constant temperature.

Preferably, the amplification of the template DNA according to the method for amplifying of the present invention takes place by means of rolling circle amplification (RCA), by means of multiple detachment amplification (MDA), strand displacement amplification (SDA) or loop mediated amplification (LAMPA).

It is also disclosed a kit or device comprising elements suitable for carrying out the method for replicating, amplifying or sequencing of the present invention.

It is also disclosed a kit for carrying out the method for replicating, amplifying or sequencing of the present invention comprising:
a) a φ29 type DNA polymerase,
b) polyoxyethylenated sorbitan monolaurate,
c) an ammonium salt,
d) a buffer, and
e) magnesium chloride.

Preferably, said ammonium salt is selected from the list comprising: ammonium sulfate, ammonium chloride or ammonium acetate.

The kit disclosed herein may further comprise a potassium salt. Preferably, said potassium salt is potassium chloride or potassium acetate.

The kit disclosed herein may further comprise a primer. The primer may be an arbitrary primer which is protected against the action of 3'-5' exonucleases.

The kit disclosed herein may further comprise nucleoside triphosphates. For example, the kit further comprises deoxynucleoside triphosphates and/or a dideoxynucleoside triphosphate.

The kit disclosed herein may also comprise at least one nucleoside triphosphate or one labelled primer. The labelled nucleoside can be, for example, a deoxynucleoside triphosphate or a dideoxynucleoside triphosphate.

The kit can further include, without any form of limitation, buffers, agents to prevent contamination, etc. On the other hand, the kit can include all the supports and recipients necessary for putting it into practice and for its optimization. Preferably, the kit further comprises the instructions for carrying out the method of the invention.

Throughout the description and claims, the word "comprises" and its variants do not exclude other technical features, additives, components or steps. For the persons skilled in the art, other objects, advantages and features of the invention will be inferred in part from the description and in part from the practice of the invention. The following drawings and examples are provided by way of illustration and do not limit the present invention.

### Description of the Drawings

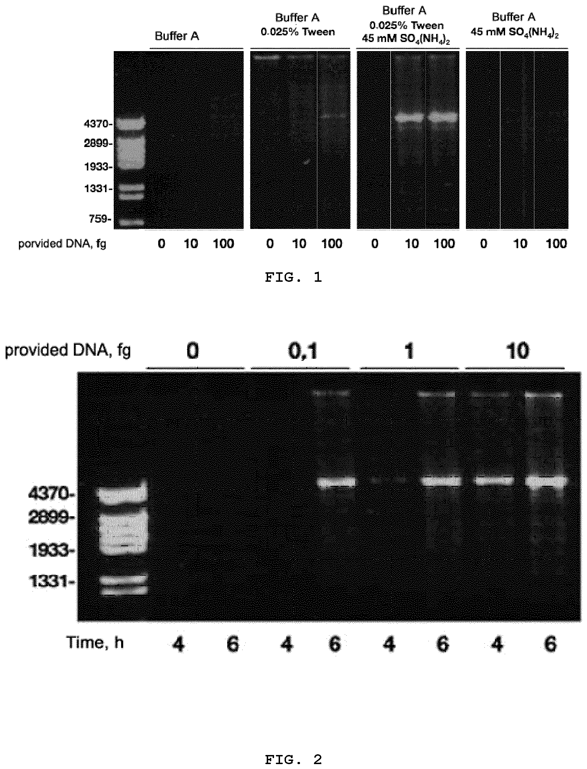

Figure 1 shows the effect of Tween^{®} 20 and (NH4)₂SO₄ in the amplification capacity of the φ29 DNA polymerase. The assay was carried out as described in the main text in the presence of the indicated amounts of plasmid DNA (4.2 kpb). After incubating at 30°C for 5 h, the reactions were analyzed as described in the main text. On the left, the linear DNA fragments obtained after digesting the φ29 DNA with *Hind*III used as DNA length markers.
Figure 2 shows the amplification of different amounts of plasmid DNA (in the order of femtograms) by the φ29 DNA polymerase in the presence of Tween^{®} 20 and (NH4)₂SO₄. The assay was carried out as described in the main text in the presence of 0.025% Tween^{®} 20 and of 45 mM (NH4)₂SO₄. The DNA length markers are the same as those used in Figure 1.

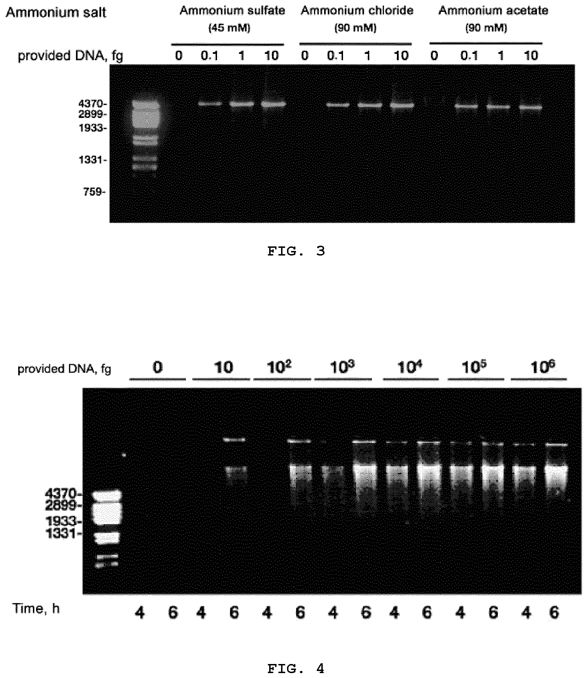

Figure 3 shows the effect of the NH₄⁺ ion in the amplification capacity of the φ29 DNA polymerase. The assay was carried out as described in the main text in the presence of 0.025% Tween^{®} 20 and the indicated ammonium salt as well as the indicated amounts of plasmid DNA (4.2 kpb). After incubating at 30°C for 6 h, the reactions were analyzed as described in the main text. The DNA length markers are the same as those used in Figure 1.
Figure 4 shows the amplification of different amounts of Bacillus subtilis genomic DNA by the φ29 DNA polymerase in the presence of Tween^{®} 20 and (NH4)₂SO₄. The assay was carried out as described in the main text in the presence of 0.025% Tween^{®} 20 and 45 mM (NH4)₂SO₄. The DNA length markers are the same as those used in Figure 1.

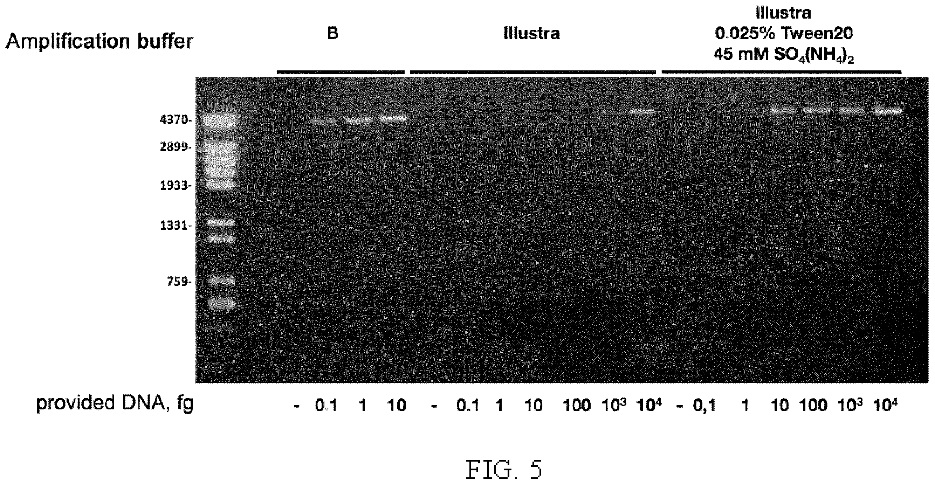

Figure 5 shows the significant improvement depicted by the addition of 0.025% Tween^{®} 20 and 45 mM (NH₄)₂SO₄ to the current reaction buffer of a commercial kit for the amplification of DNA based on the ø29 DNA polymerase (Illustra kit of General Electrics HealthCare). The assay was carried out as described in the main text. The DNA length markers are the same as those used in Figure 1.

### EXAMPLES Optimization of the experimental conditions for amplifying multiple-primer DNA by the φ29 "DNA polymerase.

The following specific examples provided in this patent document serve to illustrate the nature of the present invention. These examples are only included for illustrative purposes and must not be interpreted as limitations to the invention claimed herein. Therefore, the examples described below illustrate the invention without limiting the field of application thereof.

It has been shown that the φ29 DNA polymerase amplifies 10⁴-10⁶ times starting from several picograms of circular DNA. For this purpose a reaction buffer containing 40 mM tris-HCl, pH 7.5, 50 mM KCl and 10 mM MgCl₂ (hereinafter Buffer A) was used. After testing the influence of different detergent and salt conditions on the DNA amplification capacity of the Φ29 DNA polymerase, it is found that the simultaneous addition of 0.025% Tween® 20 and 45 mM (NH₄)₂SO4 to the Buffer A highly improve the amplification of the limited amounts of provided DNA.

Reaction conditions for amplifying plasmid DNA.- The incubation mixture contained 12.5 µl of buffer A, 50 µM of hexamers protected against the action of the 3'-5' exonuclease, 500 µM of each of the deoxynucleoside triphosphates (dCTP, dGTP, dTTP and dATP), the indicated amounts of a plasmid DNA (with a size of 4.2 kbp) and, where indicated, 45 mM (NH₄)₂SO₄ or 0.025% Tween® 20 or a combination of both was added. The DNA was denatured by incubation at 95°C for 3 minutes and subsequent cooling in ice for 5 min. The reaction was initiated upon adding 50 nM φ29 DNA polymerase and it was stopped after the incubation at 30°C by means of heating to 65°C for 10 min. To analyze the results, 1 µl samples were taken from the reactions, the amplified DNA was digested with the EcoRI restriction endonuclease and was subjected to electrophoresis in 0.7% agarose gel. The DNA was detected by means of staining the gels with ethidium bromide.

Reaction conditions for amplifying genomic DNA.- The incubation mixture contained 12.5 µl of buffer A, 45 mM (NH₄)₂SO₄, 0.025% Tween® 20, 50 µM of hexamers protected against the action of the 3'-5' exonuclease, 500 µM of each of the deoxynucleoside triphosphates (dCTP, dGTP, dTTP and dATP) and the indicated amounts of Bacillus subtilis genomic DNA (with a size of 4 Mpb). The DNA was denatured by incubation at 95°C for 3 minutes and subsequent cooling in ice for 5 min. The reaction was initiated upon adding 50 nM φ29 DNA polymerase and it was stopped after the incubation at 30°C by means of heating to 65°C for 10 min. To analyze the results, 1 µl samples were taken from the reactions and were subjected to electrophoresis in 0.7% agarose gel. The DNA was detected by means of staining the gels with ethidium bromide.

Figure 1 shows the effect of adding 45 mM (NH₄)₂SO₄ and 0.025% Tween® 20 in the amplification of small amounts of provided plasmid DNA. As shown, the Φ29 DNA polymerase did not give any amplification product detectable with the standard Buffer A when 100 fg of provided DNA were used. In these reaction conditions, the addition of 0.025% Tween® 20 in the absence of DNA caused the appearance of trace DNA products, most probably as a consequence of the nonspecific DNA amplification caused by the hybridization and elongation of the random hexamer primers. The same trace was observed with 10 fg of provided DNA. However, in the presence of 100 fg of provided DNA, the addition of 0.025% Tween® 20 allowed the Φ29 DNA polymerase to produce a detectable amount of amplified plasmid. The total production of specific or nonspecific amplified DNA indicates that the addition of 0.025% Tween® 20 to the Buffer A powers the amplification capacity of the Φ29 DNA polymerase. An effect similar with the NP40 detergent was observed. Contrarily, other analyzed detergents such as Triton X100 and Triton X114 did not power the amplification capacity of the Φ29 DNA polymerase (not shown). The simultaneous addition of 0.025% Tween® 20 and 45 mM (NH₄)₂SO₄ to the buffer A has two consequences in the yield and the specificity of the amplified products: 1) DNA amplification in the absence of provided DNA was not detected; 2) several µg of plasmid DNA of unit length were obtained by amplification even when the provided amount of DNA was as low as 10 fg. As control, the addition of 45 mM (NH4)₂SO₄ to the Buffer A did not produce any improvement in the amplification capacity of the Φ29 DNA polymerase.

Therefore, it can be concluded that the simultaneous addition of 0.025% Tween® 20 and 45 mM (NH₄)₂SO₄ to the Buffer A (hereinafter Buffer B) produces a clear optimization of the experimental conditions for carrying out the amplification with multiple priming of circular DNA by the Φ29 DNA polymerase, both being absolutely necessary reactants to amplify limited amounts (10 fg) of provided DNA. In fact, as can be seen in Figure 2, the use of Buffer B allowed the Φ29 DNA polymerase to synthesize micrograms of DNA by using a provided amount of plasmid as low as 0.1 fg (~24 molecules) after 6 hours of reaction. As quality control, the digestion of the amplification products with *EcoRI* generated linear dsDNA fragments of 4.2 kb which indicated that the amplification products were really tandem repeats of the original plasmid. Again, the Buffer B also prevented the nonspecific DNA amplification (see in Figure 2 the lanes corresponding to the reactions carried out without provided DNA).

Figure 3 shows the effect of the ammonium ions and 0.025% Tween® 20 in improving the amplification of small amounts of plasmid DNA. The assay was carried out in the previously mentioned conditions in the presence of 0.025% Tween® 20 and the indicated ammonium salt. As can be observed in Figure 3 both the NH₄Cl and the NH₄CH₃COO had a similar effect to the (NH₄)₂SO₄ both in the yield and in the specificity of the amplified products. This result indicates that the aforementioned effect of the (NH₄)₂SO₄ in the amplification of limiting amounts of plasmid DNA is due to the NH₄⁺ ions.

To determine if the optimized conditions described above also applied to the amplification of genomic DNA, the same type of assays performed in the presence of limited concentrations of *B. subtilis* genomic DNA (4 Mpb in length) was carried out. As shown in Figure 4, the presence of 0.025% Tween® 20 and 45 mM (NH₄)₂SO₄ in the buffer B, on the one hand prevented the nonspecific DNA amplification (lanes without provided DNA), and on the other hand, allowed the Φ29 DNA polymerase to give detectable and specific genomic DNA amplification even when 10 fg of provided DNA were used, i.e., an amount 10⁶ times lower than that recommended in the current commercial genomic amplification kits.

To determine if the simultaneous addition of 0.025% Tween® 20 and 45 mM (NH4)₂SO₄ increases the amplification efficiency of the current commercial kits for amplifying DNA based on the 029 DNA polymerase, the same type of plasmid DNA amplification assays described in Figures 1, 2 and 3 was carried out. Figure 5 shows the significant improvements depicted by the addition of 0.025% Tween® 20 and 45 mM (NH₄)₂SO₄ to the current reaction buffer of the Illustra kit (GE HealthCare). As can be observed, by following the recommendations of the supplier, with the Illustra kit only amounts of plasmid provided equal to or greater than 10 pg can be amplified in a detectable manner in agarose gel. Contrarily, the simultaneous addition of 0.025% Tween® 20 and 45 mM (NH₄)₂SO₄ to the reaction buffer of the Illustra kit significantly reduces the needed amount of DNA which can be amplified, amplification products from provided 1 fg of plasmid DNA being observed, involving an improvement of four orders of magnitude in the amplification.

## Claims

1. Method for replicating, amplifying or sequencing a Template DNA which comprises contacting said DNA with a reaction mixture comprising, at least:
a) a ®29 type DNA polymerase,
b) polyoxyethylenated sorbitan monolaurate, wherein the polyoxyethylenated sorbitan monolaurate is in a proportion between 0.006% and 0.05% of the total volume of the reaction,
c) an ammonium salt, wherein the ammonium salt is ammonium sulfate at a concentration between 30 mM and 60 mM, ammonium chloride at a concentration between 60 mM and 120 mM or ammonium acetate at a concentration between 60 mM and 120 mM,
d) a buffer,
e) magnesium chloride,
f) a primer, and
g) nucleoside triphosphates.

2. The method according to claim 1, wherein the reaction mixture further comprises a potassium salt.

3. The method according to claim 2, wherein the potassium salt is potassium chloride or potassium acetate.

4. The method according to claim 1, wherein the polyoxyethylenated sorbitan monolaurate is in a proportion between 0.01% and 0.03% of the total volume of the reaction.

5. The method according to any of claims 1 to 4, wherein the ammonium sulfate is at a concentration between 40 mM and 50 mM.

6. The method according to any of claims 1 to 4, wherein the ammonium chloride or the ammonium acetate is at a concentration between 80 mM and 100 mM.

7. The method according to any of claims 1 to 6, wherein the Φ 29 type DNA polymerase is selected from the DNA polymerases isolated from the following phages: Φ 29, Cp-1, PRD-1, Φ 15, Φ 21, PZE, PZA, Nf, M2Y, B103, GA-1, SF5, Cp-5, Cp-7, PR4, PR5, PR722, L17 or ABV.

8. The method according to claim 7, wherein the Φ 29 type DNA polymerase has an amino acid sequence having an identity of at least 80% with the SEQ ID NO: 1.

9. The method according to claim 8, wherein the Φ 29 type DNA polymerase has an amino acid sequence having an identity of at least 90% with the SEQ ID NO: 1.

10. The method according to claim 9, wherein the Φ 29 type DNA polymerase has the amino acid sequence SEQ ID NO: 1.

11. The method according to any of claims 1 to 9, wherein the Φ 29 type DNA polymerase has a modification in the exonuclease domain and wherein said modified DNA polymerase has less than 10 % of exonuclease activity than the corresponding naturally occurring DNA polymerase.

12. The method according to claim 11, wherein the modified Φ 29 type DNA polymerase has less than 1% of exonuclease activity than the corresponding naturally occurring DNA polymerase.

13. The method according to claim 12, wherein the modified Φ 29 type DNA polymerase lacks detectable exonuclease activity with respect to the corresponding naturally occurring DNA polymerase.

14. The method according to any of claims 1 to 13, wherein the buffer is tris-hydrochloric, tris-acetic or HEPES.

15. The method according to any of claims 1 to 14, wherein the buffer is at a pH between 7 and 8.5.

16. The method according to any of claims 1 to 15, wherein the magnesium chloride is at a concentration between 2 mM and 20 mM.

17. The method according to claim 16, wherein the magnesium chloride is at a concentration between 5 mM and 15 mM.

18. The method according to any of claims 2 to 17, wherein the potassium chloride or the potassium acetate is at a concentration between 30 mM and 70 mM.

19. The method according to claim 18, wherein the potassium chloride or the potassium acetate is at a concentration between 40 mM and 60 mM.

20. The method according to any of claims 1 to 19, wherein the nucleoside triphosphates are dCTP, dGTP, dTTP and dATP.

21. The method according to claim 20, wherein the dCTP, dGTP, dTTP and dATP nucleoside triphosphates are in equimolar amounts.

22. The method according to any of claims 1 to 21, wherein the primer is arbitrary and is protected against the action of exonucleases.

23. The method according to any of claims 1 to 22, wherein the template DNA is plasmid DNA.

24. The method according to any of claims 1 to 22, wherein the template DNA is genomic DNA.

25. The method according to any of claims 1 to 24, wherein the amplification is performed at an essentially constant temperature between 25 and 40 °C.

26. The method for amplifying a template DNA according to any of claims 1 to 25, wherein the amplification takes place by means of rolling circle amplification (RCA), multiple displacement amplification (MDA), strand displacement amplification (SDA) or loop mediated amplification (LAMPA).

27. The method according to any of claims 1 to 26, wherein at least one nucleoside triphosphate or one primer is labelled.

## Patentansprüche

1. Verfahren zum Replizieren, Amplifizieren oder Sequenzieren einer Matrizen-DNA, welches das in Kontakt bringen der DNA mit einem Reaktionsgemisch umfasst, das zumindest umfasst:
a) eine Φ29-Typ DNA-Polymerase,
b) polyoxyethyleniertes Sorbitanmonolaurat, wobei das polyoxyethylenierte Sorbitanmonolaurat in einem Anteil zwischen 0,006 % und 0,05 % des Gesamtvolumens der Reaktion vorliegt,
c) ein Ammoniumsalz, wobei das Ammoniumsalz Ammoniumsulfat mit einer Konzentration zwischen 30 mM und 60 mM, Ammoniumchlorid mit einer Konzentration zwischen 60 mM und 120 mM oder Ammoniumacetat mit einer Konzentration zwischen 60 mM und 120 mM ist,
d) ein Puffer,
e) Magnesiumchlorid,
f) ein Primer, und
g) Nukleosidtriphosphate.

2. Verfahren nach Anspruch 1, wobei das Reaktionsgemisch weiterhin ein Kaliumsalz umfasst.

3. Verfahren nach Anspruch 2, wobei das Kaliumsalz Kaliumchlorid oder Kaliumacetat ist.

4. Verfahren nach Anspruch 1, wobei das polyoxyethylenierte Sorbitanmonolaurat in einem Anteil zwischen 0,01 % und 0,03 % des Gesamtvolumens der Reaktion vorliegt.

5. Verfahren nach einem der Ansprüche 1 bis 4, wobei das Ammoniumsulfat mit einer Konzentration zwischen 40 mM und 50 mM vorliegt.

6. Verfahren nach einem der Ansprüche 1 bis 4, wobei das Ammoniumchlorid oder das Ammoniumacetat mit einer Konzentration zwischen 80 mM und 100 mM vorliegt.

7. Verfahren nach einem der Ansprüche 1 bis 6, wobei die Φ29-Typ DNA-Polymerase aus den DNA-Polymerasen ausgewählt wird, die aus den folgenden Phagen isoliert wird: Φ 29, Cp-1, PRD-1, Φ 15, Φ 21, PZE, PZA, Nf, M2Y, B103, GA-1, SF5, Cp-5, Cp-7, PR4, PR5, PR722, L17 oder ABV.

8. Verfahren nach Anspruch 7, wobei die Φ 29-Typ DNA-Polymerase eine Aminosäuresequenz hat, die zumindest 80 % Identität mit der SEQ ID NO: 1 hat.

9. Verfahren nach Anspruch 8, wobei die Φ 29-Typ DNA-Polymerase eine Aminosäuresequenz hat, die zumindest 90 % Identität mit der SEQ ID NO: 1 hat.

10. Verfahren nach Anspruch 9, wobei die Φ 29-Typ DNA-Polymerase die Aminosäuresequenz SEQ ID NO: 1 hat.

11. Verfahren nach einem der Ansprüche 1 bis 9, wobei die Φ 29-Typ DNA-Polymerase eine Modifikation in der Exonukleasedomäne hat und wobei die modifizierte DNA-Polymerase weniger als 10 % der Exonukleaseaktivität der entsprechenden natürlich vorkommenden DNA-Polymerase hat.

12. Verfahren nach Anspruch 11, wobei die modifizierte Φ 29-Typ DNA-Polymerase weniger als 1 % der Exonukleaseaktivität der entsprechenden natürlich vorkommenden DNA-Polymerase hat.

13. Verfahren nach Anspruch 12, wobei die modifizierte Φ 29-Typ DNA-Polymerase keine detektierbare Exonukleaseaktivität in Bezug auf die entsprechende natürlich vorkommende DNA-Polymerase aufweist.

14. Verfahren nach einem der Ansprüche 1 bis 13, wobei der Puffer Tris-Hydrochlorid, Tris-Acetat oder HEPES ist.

15. Verfahren nach einem der Ansprüche 1 bis 14, wobei der Puffer mit einem pH zwischen 7 und 8,5 vorliegt.

16. Verfahren nach einem der Ansprüche 1 bis 15, wobei das Magnesiumchlorid mit einer Konzentration zwischen 2 mM und 20 mM vorliegt.

17. Verfahren nach Anspruch 16, wobei das Magnesiumchlorid mit einer Konzentration zwischen 5 mM und 15 mM vorliegt.

18. Verfahren nach einem der Ansprüche 2 bis 17, wobei das Kaliumchlorid oder das Kaliumacetat mit einer Konzentration zwischen 30 mM und 70 mM vorliegt.

19. Verfahren nach Anspruch 18, wobei das Kaliumchlorid oder das Kaliumacetat mit einer Konzentration zwischen 40 mM und 60 mM vorliegt.

20. Verfahren nach einem der Ansprüche 1 bis 19, wobei die Nukleosidtriphosphate dCTP, dGTP, dTTP und dATP sind.

21. Verfahren nach Anspruch 20, wobei die dCTP, dGTP, dTTP und dATP Nukleosidtriphosphate in äquimolaren Mengen vorliegen.

22. Verfahren nach einem der Ansprüche 1 bis 21, wobei der Primer beliebig ist und gegen die Aktivität von Exonukleasen geschützt ist.

23. Verfahren nach einem der Ansprüche 1 bis 22, wobei die Matrizen-DNA Plasmid-"DNA ist.

24. Verfahren nach einem der Ansprüche 1 bis 22, wobei die Matrizen-DNA genomische "DNA ist.

25. Verfahren nach einem der Ansprüche 1 bis 24, wobei die Amplifikation bei einer im Wesentlichen konstanten Temperatur zwischen 25 und 40 °C durchgeführt wird.

26. Verfahren zum Amplifizieren einer Matrizen-DNA nach einem der Ansprüche 1 bis 25, wobei die Amplifikation mittels rollender Kreis Amplifikation (engl.: *rolling circle amplification*; RCA), multiple-Dislokations-Amplifikation (engl.: *multiple displacement amplification*; MDA), Strang-Dislokations-Amplifikation (engl.: *strand displacement amplification*; SDA) oder Schlingen-vermittelte-Amplifikation (engl.: *loop mediated amplification*; LAMPA) erfolgt.

27. Verfahren nach einem der Ansprüche 1 bis 26, wobei zumindest ein Nukleosidtriphosphat oder ein Primer markiert ist.

## Revendications

1. Procédé de réplication, d'amplification ou de séquençage d'un ADN matrice qui comprend la mise en contact dudit ADN avec un mélange de réaction comprenant, au moins :
a) une ADN polymérase de type Φ 29,
b) du monolaurate de sorbitan polyoxyéthylèné, où le monolaurate de sorbitan polyoxyéthylèné est présent dans une proportion entre 0,006 % et 0,05 % du volume total de la réaction,
c) un sel d'ammonium, où le sel d'ammonium est du sulfate d'ammonium à une concentration entre 30 mM et 60 mM, du chlorure d'ammonium à une concentration entre 60 mM et 120 mM ou de l'acétate d'ammonium à une concentration entre 60 mM et 120 mM,
d) un tampon,
e) du chlorure de magnésium,
f) une amorce, et
g) des nucléosides triphosphates.

2. Le procédé selon la revendication 1, où le mélange de réaction comprend en outre un sel de potassium.

3. Le procédé selon la revendication 2, où le sel de potassium est du chlorure de potassium ou de l'acétate de potassium.

4. Le procédé selon la revendication 1, où le monolaurate de sorbitan polyoxyéthylèné est présent dans une proportion entre 0,01 % et 0,03 % du volume total de la réaction.

5. Le procédé selon l'une quelconque des revendications 1 à 4, où le sulfate d'ammonium est à une concentration entre 40 mM et 50 mM.

6. Le procédé selon l'une quelconque des revendications 1 à 4, où le chlorure d'ammonium ou l'acétate d'ammonium est à une concentration entre 80 mM et 100 mM.

7. Le procédé selon l'une quelconque des revendications 1 à 6, où l'ADN polymérase de type Φ 29 est sélectionnée parmi les ADN polymérases isolées à partir des phages suivants : Φ 29, Cp-1, PRD-1, Φ 15, Φ 21, PZE, PZA, Nf, M2Y, B103, GA-1, SF5, Cp-5, Cp-7, PR4, PR5, PR722, L17 ou ABV.

8. Le procédé selon la revendication 7, où l'ADN polymérase de type Φ 29 a une séquence d'acides aminés présentant une identité d'au moins 80% avec la SEQ ID NO: 1.

9. Le procédé selon la revendication 8, où l'ADN polymérase de type Φ 29 a une séquence d'acides aminés présentant une identité d'au moins 90% avec la SEQ ID NO: 1.

10. Le procédé selon la revendication 9, où l'ADN polymérase de type Φ 29 a une séquence d'acides aminés SEQ ID NO: 1.

11. Le procédé selon l'une quelconque des revendications 1 à 9, où l'ADN polymérase de type Φ29 présente une modification dans le domaine exonucléase et où ladite ADN polymérase modifiée présente moins de 10 % de l'activité exonucléase de l'ADN polymérase d'origine naturelle correspondante.

12. Le procédé selon la revendication 11, où l'ADN polymérase de type Φ 29 modifiée présente moins de 1 % de l'activité exonucléase de l'ADN polymérase d'origine naturelle correspondante.

13. Le procédé selon la revendication 12, où l'ADN polymérase de type Φ 29 modifiée ne possède pas d'activité exonucléase détectable par rapport à l'ADN polymérase d'origine naturelle correspondante.

14. Le procédé selon l'une quelconque des revendications 1 à 13, où le tampon est tris-acide chlorhydrique, tris-acide acétique ou HEPES.

15. Le procédé selon l'une quelconque des revendications 1 à 14, où le tampon est à un pH entre 7 et 8,5.

16. Le procédé selon l'une quelconque des revendications 1 à 15, où le chlorure de magnésium est à une concentration entre 2 mM et 20 mM.

17. Le procédé selon la revendication 16, où le chlorure de magnésium est à une concentration entre 5 mM et 15 mM.

18. Le procédé selon l'une quelconque des revendications 2 à 17, où le chlorure de potassium ou l'acétate de potassium est à une concentration entre 30 mM et 70 mM.

19. Le procédé selon la revendication 18, où le chlorure de potassium ou l'acétate de potassium est à une concentration entre 40 mM et 60 mM.

20. Le procédé selon l'une quelconque des revendications 1 à 19, où les nucléosides triphosphates sont dCTP, dGTP, dTTP et dATP.

21. Le procédé selon la revendication 20, où les nucléosides triphosphates dCTP, dGTP, dTTP et dATP sont en quantités équimolaires.

22. Le procédé selon l'une quelconque des revendications 1 à 21, où l'amorce est arbitraire et est protégée contre l'action des exonucléases.

23. Le procédé selon l'une quelconque des revendications 1 à 22, où l'ADN matrice est un ADN plasmidique.

24. Le procédé selon l'une quelconque des revendications 1 à 22, où l'ADN matrice est un ADN génomique.

25. Le procédé selon l'une quelconque des revendications 1 à 24, où l'amplification est effectuée à une température essentiellement constante entre 25 et 40 °C.

26. Le procédé d'amplification d'un ADN matrice selon l'une quelconque des revendications 1 à 25, où l'amplification est effectuée au moyen d'une amplification par cercle roulant (RCA), une amplification par déplacement multiple (MDA), une amplification par déplacement de brin (SDA) ou une amplification médiée par boucle (LAMPA).

27. Le procédé selon l'une quelconque des revendications 1 à 26, où au moins un nucléoside triphosphate ou une amorce est marqué.
